(19) Europäisches Patentamt
European Patent Office
Office européen des brevets

(11) **EP 2 425 873 A1**

(12) **DEMANDE DE BREVET EUROPEEN**

(43) Date de publication:
**07.03.2012 Bulletin 2012/10**

(51) Int Cl.:
***A61N 5/06*** *(2006.01)*

(21) Numéro de dépôt: **10305960.6**

(22) Date de dépôt: **07.09.2010**

(84) Etats contractants désignés:
**AL AT BE BG CH CY CZ DE DK EE ES FI FR GB
GR HR HU IE IS IT LI LT LU LV MC MK MT NL NO
PL PT RO SE SI SK SM TR**
Etats d'extension désignés:
**BA ME RS**

(71) Demandeur: **Steba Maor SA
2613 Luxemburg (LU)**

(72) Inventeurs:
• **Betrouni, Nacim
59370 Mons En Baroeul (FR)**

• **Hardy, Joseph
75017 Paris (FR)**
• **Boukris, Stephan
75016 Paris (FR)**

(74) Mandataire: **L'Helgoualch, Jean et al
Cabinet Sueur et L'Helgoualch
Le Centralis
63, avenue du Général Leclerc
92340 Bourg-la-Reine (FR)**

(54) **Modélisation de l'action d'une fibre optique dans un traitement par therapie photodynamique, et assistance à la planification d'un tel traitement**

(57)    L'invention concerne un procédé (200) d'assistance à la planification d'un traitement par thérapie photodynamique d'un patient, lors duquel une substance photosensible prédéfinie doit être administrée au patient, puis soumise à une illumination à une longueur d'onde prédéterminée par l'intermédiaire d'un nombre de fibres optiques aptes à être introduites sur une longueur d'insertion dans la zone à traiter selon une position par rapport à une grille de brachythérapie. Le procédé réalise une mesure (230) du volume de la zone à traiter par reconstruction volumique à partir d'un traitement numérique de contours saisis directement sur une série d'images numériques de la zone à traiter, puis une détermination (250) par le calcul du nombre de fibres optiques à utiliser, de leur position par rapport à la grille de brachythérapie et de leur longueur d'insertion qui optimisent la correspondance d'un volume total théorique d'action calculé avec le volume mesuré de la zone à traiter, le volume total théorique d'action étant calculé en fonction de la position de chaque fibre, et d'un volume élémentaire d'action théorique d'une fibre correspondant au volume d'un cylindre de rayon d'action R prédéterminé, et de hauteur correspondant à la longueur d'insertion de la fibre.

L'invention concerne également un procédé (100) de modélisation du rayon d'action d'une fibre optique utilisant une corrélation de volumes mesurés de zones effectivement nécrosées suite à une pluralité d'essais cliniques menés sur différents patients avec des volumes théoriques calculés à partir des mêmes ensembles de paramètres que ceux utilisés dans les essais cliniques et du volume élémentaire d'action théorique d'une fibre.

FIG.9

## Description

La présente invention concerne un procédé de modélisation de l'action d'une fibre optique destinée à être utilisée dans un traitement d'un patient par thérapie photodynamique, et un procédé d'assistance à la planification d'un tel traitement.

La thérapie photodynamique, ou PDT (initiales anglo-saxonnes mises pour PhotoDynamic Therapy), est une thérapie consistant à administrer une substance photosensible, qui se fixe préférentiellement dans les tissus, par exemple sur les cellules tumorales, et à irradier ensuite les tissus au moyen d'une source lumineuse de longueur d'onde appropriée, susceptible d'activer la substance photosensible, qui libère ainsi in situ de l'oxygène singulet ou des radicaux libres qui sont extrêmement réactifs et oxydent les tissus immédiatement voisins, provoquant la mort des cellules cancéreuses par apoptose (mort cellulaire programmée) ou par ischémie en ciblant les vaisseaux sanguins irriguant les cellules tumorales (technique de Vascular-Targeted Photodynamic Therapy ou VTP). Les espèces radicalaires oxygénées ainsi produites ont généralement une faible diffusion et une très courte durée de vie si bien que leur effet toxique est très localisé. La technique de la PDT permet de traiter des pathologies comme certains cancers ou la DMLA (Dégénérescence Maculaire Liée à l'Age).

Récemment, plusieurs études ont montré que la PDT était une alternative efficace pour le traitement du cancer de la prostate, en associant la substance photosensible à des lasers spécifiques et des fibres optiques dédiées.

C'est ainsi que la Société Demanderesse a étudié divers agents photosensibles, notamment le WST-09, ou Tookad®, et plus récemment le WST-11, décrits dans les demandes de brevet WO 2004045492 et EP 1137411, qui se sont révélés particulièrement adaptés au traitement du cancer de la prostate.

Dans le cas du cancer de la prostate, le produit photosensible est tout d'abord administré par voie intraveineuse au patient afin d'être capté par les cellules cancéreuses. A ce stade, le médicament demeure inactif tant qu'il n'a pas été exposé à la lumière à la longueur d'onde appropriée.

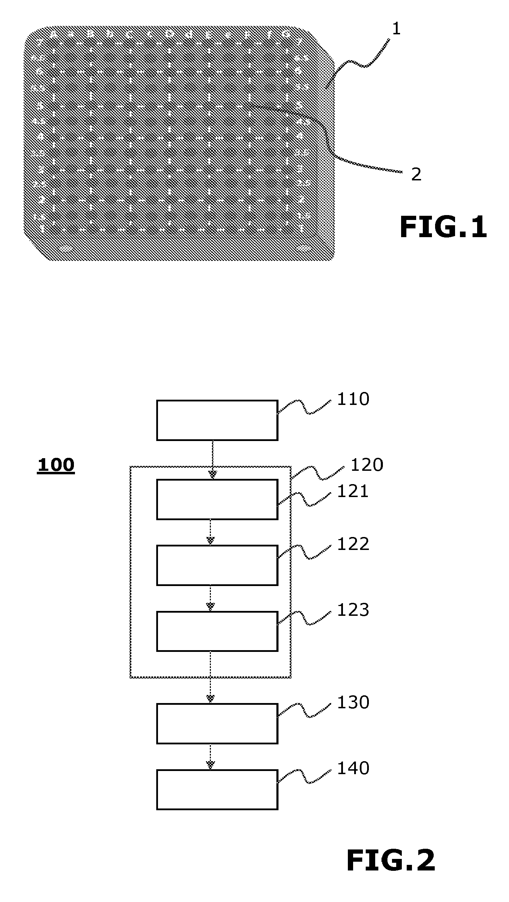

La lumière est ensuite appliquée par l'intermédiaire d'un laser alimentant plusieurs fibres optiques positionnées sous guidage échographique. Pour ce faire, plusieurs fibres optiques aptes à être alimentées par un laser sont introduites dans la prostate. Une grille externe de type de celle utilisée en brachythérapie permet au chirurgien de positionner précisément les différentes fibres optiques au sein de la prostate et les unes par rapport aux autres. Plus précisément, une telle grille comporte une pluralité d'orifices arrangés dans un même plan selon une matrice de plusieurs lignes et de plusieurs colonnes d'écartement connu. A titre d'exemple, la grille 1 de brachythérapie représentée sur la figure 1 comporte une matrice de treize lignes et de treize colonnes espacées de 0,5 mm, avec un orifice traversant placé à chaque intersection d'une ligne et d'une colonne, comme l'orifice 2 situé à l'intersection de la colonne « F » et de la ligne « 5 ». Chaque fibre optique (non représentée sur la figure 1) est introduite perpendiculairement à la grille externe au travers d'un orifice jusqu'à pénétrer dans la zone à traiter de la prostate. Un guidage échographique au moyen d'une sonde reliée à un moniteur de contrôle, permet au chirurgien de visualiser la prostate sur l'écran du moniteur et de faire pénétrer chaque fibre sur une longueur de pénétration donnée.

Pour un traitement efficace, le nombre de fibres utilisées, leur positionnement dans un orifice particulier de la grille de brachythérapie, et la longueur d'introduction de chaque fibre dans la zone à traiter doivent être très précisément déterminés pour chaque patient. En particulier dans le cas du traitement de la prostate, la planification de traitement individuelle est essentielle car les différents paramètres que constituent le nombre de fibres optiques, leur positionnement, et leur longueur d'introduction vont clairement varier d'un patient à un autre en fonction de la nature de la prostate (volume, forme,..), de la localisation des tumeurs cancéreuses, et des choix thérapeutiques (traitement focal, hémiablation..).

On connaît déjà, notamment du document « Treatment and planning and dose analysis for interstitial photodynamic therapy of prostate cancer », Sean R H Davidson et al, Phys. Med. Biol. 54 (2009) 2293-2313, un produit logiciel pour la mise en oeuvre d'un procédé d'assistance à la planification d'un traitement par thérapie photodynamique de la prostate dans le but de garantir qu'un patient va recevoir la dose suffisante d'illumination pour la zone ciblée ou zone à traiter, tout en minimisant la dose d'illumination reçue par les zones environnantes non ciblées. Dans ce document, la planification est fondée sur la prédiction de la distribution de lumière dans la prostate et les zones environnantes, et plus précisément sur une résolution de l'équation de la diffusion de la lumière par une méthode de résolution à éléments finis. L'action des fibres optiques associées à la substance photosensible ainsi modélisée, la planification consiste alors à rechercher la configuration (notamment le nombre de fibres et le positionnement des fibres par rapport à une grille de brachythérapie) pour laquelle la distribution de lumière modélisée est la mieux appropriée au traitement du patient.

L'inconvénient principal de cette méthode de planification réside dans les calculs mathématiques complexes intervenant dans la modélisation. De ce fait, la durée totale pour obtenir les résultats de la planification, fonction des paramètres de la configuration, peut dépasser plusieurs heures. Les ajustements effectués par les praticiens (radiologues ou chirurgiens) doivent être effectués itérativement et manuellement, jusqu'à l'obtention d'une distribution de lumière compatible avec celle recherchée.

Cette méthode rencontre des difficultés d'application marquées : L'introduction d'une sonde, puis des fibres optiques au sein de la prostate modifient de façon significative la forme et le volume de la prostate, et impactent la cohérence du plan, le modèle privilégiant la position relative des fibres optiques au regard de la cible (zone à traiter).

Du fait du temps nécessaire au calcul, un ajustement aux paramètres effectivement relevés au moment du traitement (par la sonde échographique) s'avère irréalisable.

Ainsi, il n'existe à ce jour aucune méthode connue de modélisation de l'action des fibres optiques ni d'assistance à la planification d'un traitement PDT qui soit suffisamment simple pour délivrer quasiment automatiquement une configuration optimisée par rapport à chaque patient des paramètres à appliquer lors d'un traitement PDT, et qui ne nécessite que peu d'interventions du praticien.

La présente invention a notamment pour but de pallier les inconvénients des méthodes connues en proposant d'une part, un outil de modélisation mis en oeuvre par ordinateur, fondé sur des calculs simples, et d'autre part, un outil de planification, également mis en oeuvre par ordinateur, qui soit simple d'utilisation pour le praticien, et qui permette d'obtenir très rapidement, typiquement en quelques minutes, une planification adaptée à chaque patient fournissant au praticien l'optimisation du nombre de fibres à utiliser, ainsi que leurs longueurs et leurs positions (par rapport à une grille externe comme celle utilisée en brachythérapie).

Pour ce faire, la présente invention a pour objet un procédé de modélisation, mis en oeuvre par ordinateur, de l'action d'une fibre optique destinée à être utilisée dans un traitement d'un patient par thérapie photodynamique pour illuminer, à une longueur d'onde prédéfinie, une zone à traiter sur une longueur d'insertion de la fibre optique à l'intérieur de ladite zone à traiter, afin d'activer une substance photosensible prédéfinie préalablement administrée audit patient et présente dans ladite zone à traiter, caractérisé en ce qu'il consiste à modéliser le volume élémentaire d'action théorique de la fibre optique par celui d'un cylindre de rayon d'action $R$ et de longueur $L$ correspondant à ladite longueur d'insertion, et à déterminer ledit rayon d'action $R$ par corrélation de volumes mesurés de zones effectivement nécrosées suite à une pluralité d'essais cliniques menés sur différents patients en utilisant ladite substance photosensible associée à au moins une fibre optique, chaque essai clinique étant associé à un ensemble de paramètres correspondant aux conditions réelles de l'essai clinique et comprenant au moins le nombre de fibres optiques utilisées, leur position par rapport à une grille de brachythérapie, et la longueur d'insertion de chacune des fibres dans la zone à traiter, avec des volumes d'action théoriques calculés à partir du même ensemble de paramètres et du volume élémentaire d'action théorique.

Le procédé de modélisation comporte de préférence les étapes suivantes :

- Construction d'une base de données informatique à partir desdits essais cliniques, par mémorisation pour chaque patient, dans ladite base de données, d'un premier fichier numérique correspondant à une série d'images numériques de la zone à traiter avant l'essai clinique, d'un deuxième fichier numérique correspondant à une série d'images numériques de la zone après l'essai clinique, et dudit ensemble de paramètres correspondant aux conditions réelles de l'essai clinique ;

- Mesure, pour chaque patient de la base de données, du volume de la zone effectivement nécrosée lors de l'essai clinique, à partir desdits premier et deuxième fichiers numériques ;

- Calcul, pour chaque patient de la base de données, du volume total théorique d'action en fonction des paramètres dudit ensemble et du volume élémentaire d'action théorique d'une fibre;

- Détermination dudit rayon d'action $R$ d'une fibre optique par corrélation du volume total théorique d'action calculé, pour chaque patient dans la base de données, avec le volume mesuré de la zone effectivement nécrosée.

Les séries d'images numériques desdits premier et deuxième fichiers numériques correspondent de préférence à des images transversales de la zone respectivement avant et après traitement.

Les images numériques sont par exemple des images à résonance magnétique ou des images par ultrasons.

Dans une mise en oeuvre préférée, l'étape de mesure du volume de la zone effectivement nécrosée comporte les étapes suivantes :

- Chargement et affichage de la série d'images du deuxième fichier informatique sur une interface graphique utilisateur affichée sur un écran de l'ordinateur;

- Contourage de la zone effectivement nécrosée par saisie directe sur chaque image de la série affichée sur l'écran d'ordinateur;

- Mesure du volume de la zone effectivement nécrosée par reconstruction volumique à partir d'un traitement numérique des contours saisis.

La présente invention a également pour objet un procédé d'assistance à la planification d'un traitement d'un patient par thérapie photodynamique utilisant les mêmes principes de modélisation que ceux du procédé de modélisation ci-dessus. Plus précisément, un procédé d'assistance, mis en oeuvre par ordinateur, à la planification d'un traitement d'un patient par thérapie photodynamique, lors duquel une substance photosensible prédéfinie doit être administrée au patient, puis soumise à une illumination à une longueur d'onde prédéterminée par l'intermédiaire d'un nombre de fibres optiques aptes à être introduites sur une longueur d'insertion dans la zone à traiter selon une position par rapport à une grille de brachythérapie, est caractérisé en ce qu'il comporte les étapes suivantes:

- Chargement et affichage d'un fichier numérique correspondant à une série d'images numériques de la zone à traiter sur une interface graphique utilisateur affichée sur un écran de l'ordinateur;

- Contourage de la zone à traiter par saisie directe sur chaque image de la série affichée sur l'écran d'ordinateur;

- Mesure du volume de la zone à traiter par reconstruction volumique à partir d'un traitement numérique des contours saisis ;

- Affichage et positionnement d'une représentation plane de la grille de brachythérapie en surimpression sur chaque image de la série et contours saisis correspondants ;

- Détermination par le calcul du nombre de fibres optiques à utiliser, de leur position par rapport à la grille de brachythérapie et de leur longueur d'insertion qui optimisent la correspondance d'un volume total théorique d'action calculé avec le volume mesuré de la zone à traiter, ledit volume total théorique d'action étant calculé en fonction de la position de chaque fibre, et d'un volume élémentaire d'action théorique d'une fibre correspondant au volume d'un cylindre de rayon d'action R prédéterminé, et de hauteur correspondant à la longueur d'insertion de la fibre.

Le rayon d'action R est de préférence prédéterminé conformément au procédé de modélisation.
En variante, le rayon d'action R est sélectionné parmi un ensemble de valeurs possibles.
Selon une mise en oeuvre préférée du procédé d'assistance selon l'invention, l'étape de détermination par le calcul utilise un algorithme d'optimisation de type descente de gradient, par exemple l'algorithme de Powell.
L'invention a également pour objet un produit programme d'ordinateur qui, lorsqu'il est mis en oeuvre sur un ordinateur informatique, réalise le procédé de modélisation selon l'invention.
L'invention a enfin pour objet un produit programme d'ordinateur qui, lorsqu'il est mis en oeuvre sur un ordinateur informatique, réalise le procédé d'assistance à la planification selon l'invention. Les deux produits programmes d'ordinateur peuvent être indépendants, ou réunis au sein d'un même produit programme d'ordinateur.
Les différents aspects de l'invention seront mieux compris au vu de la description suivante, faite en référence aux figures annexées dans lesquelles :

- la figure 1, déjà décrite ci-dessus, représente un exemple de grille externe de type brachythérapie, servant au positionnement d'une pluralité de fibres optiques pour un traitement PDT ;

- la figure 2 illustre, sous forme de synoptique simplifié, les différentes étapes mises en oeuvre dans un procédé de modélisation conforme à l'invention;

- la figure 3 illustre les profils d'émission d'une fibre optique particulière, sous différents angles d'observation, à une longueur d'onde de 763 nm ;

- la figure 4 représente schématiquement un exemple de coupe transversale d'une prostate avant traitement, et du positionnement des fibres optiques qui ont été effectivement utilisées pour le traitement PDT lors d'un essai clinique;

- la figure 5 représente un exemple d'image à résonance magnétique montrant une coupe transversale de la prostate, obtenue sept jours après le traitement PDT d'un essai clinique ;

- la figure 6 illustre schématiquement le principe de la modélisation de l'action d'un ensemble de fibres optiques en fonction de leurs positions relatives sur une grille de brachythérapie;

- la figure 7 illustre les résultats de différentes corrélations effectuées pour valider la modélisation ;

- la figure 8 illustre schématiquement deux fibres optiques positionnées de manière à avoir une zone de recouvrement ;

- la figure 9 représente, sous forme de synoptique simplifié, différentes étapes mises en oeuvre dans un procédé d'assistance à la planification conforme à l'invention;

- la figure 10 est une copie d'écran d'ordinateur représentant une interface graphique utilisateur à l'issue d'une première étape du procédé d'assistance à la planification selon l'invention ;

- les figures 11, 12a et 12b sont des copies d'écran représentant des exemples d'affichage dans une zone de visualisation de l'interface graphique utilisateur à différentes étapes du procédé d'assistance à la planification selon l'invention.

Dans un premier temps, les différentes étapes du procédé de modélisation selon l'invention de l'action d'une fibre optique destinée à être utilisée dans un traitement anti cancéreux d'un patient par thérapie photodynamique vont être détaillées en référence à la figure 2. On rappelle également que ce procédé est destiné à être mis en oeuvre par un produit logiciel apte à être installé sur un ordinateur. Le produit logiciel comprend ainsi plusieurs routines logicielles, certaines concernant des étapes de calcul ou d'estimation intervenant dans la modélisation, d'autres étant plus spécifiquement liées à la gestion d'une interface graphique avec l'utilisateur, apte à être affichée sur l'écran de l'ordinateur, et au traitement d'informations susceptibles d'être saisies par l'utilisateur via cette interface graphique.
Dans son étude, la Demanderesse a validé son procédé de modélisation pour l'utilisation d'une fibre optique spécifique à la longueur d'onde de 763 nm, en association avec l'agent photosensible WST11, pour le traitement de la prostate. On comprendra aisément que les

4

principes de la modélisation, ainsi que ceux de l'assistance à la planification qui découlent de cette modélisation, peuvent être utilisés pour d'autres associations de fibres optiques et d'agents photosensibles, et appliqués au traitement d'autres organes.

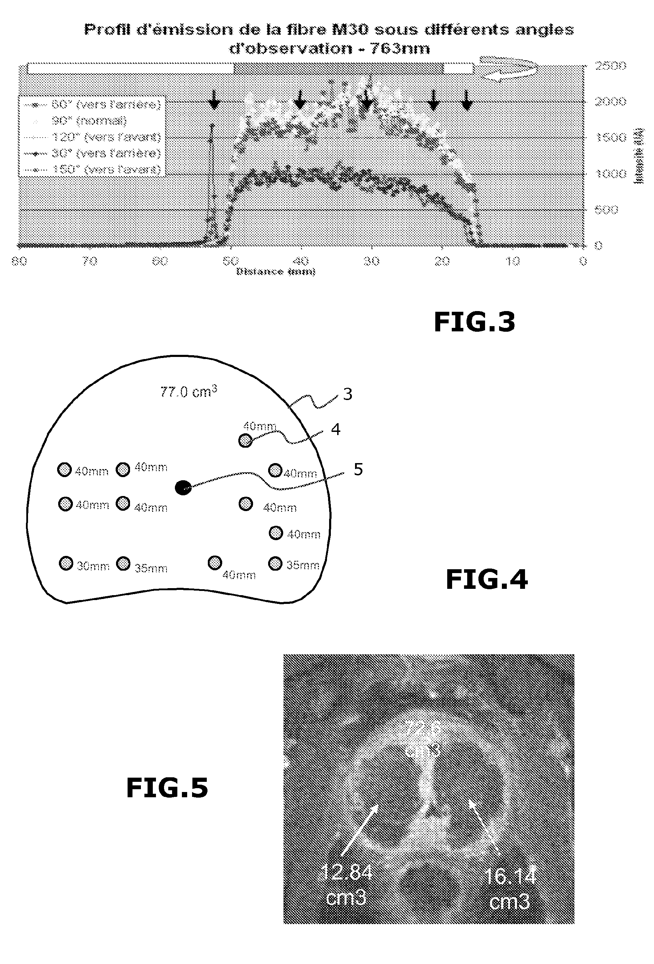

Le procédé de modélisation selon l'invention est fondé essentiellement sur le fait que le volume élémentaire d'action théorique de la fibre optique considérée en association avec l'agent photosensible peut être modélisé par celui d'un cylindre de rayon R correspondant au rayon d'action de la fibre, et de longueur L correspondant à la longueur sur laquelle la fibre émet de la lumière. Cette modélisation repose sur l'examen des profils d'émission de la fibre sous différents angles d'observation, représentés sur la figure 3. On constate en effet en comparant les différentes courbes correspondant à différents angles d'observation, que l'intensité maximale est obtenue pour un angle d'observation de 90° par rapport à l'axe longitudinal de la fibre. En conséquence, la Demanderesse a recherché un moyen de pouvoir déterminer une valeur du rayon d'action R d'une fibre, à partir des résultats d'essais cliniques antérieurs.

Les études de la Demanderesse ont montré qu'il était possible d'établir une relation affine, avec un coefficient de corrélation supérieur à 0,8, entre :

- d'une part, les volumes de zones effectivement nécrosées lors d'essais cliniques menés sur différents patients en utilisant une même substance photosensible associée à au moins une fibre optique, chaque essai clinique étant associé à un ensemble de paramètres correspondant aux conditions réelles de l'essai clinique et comprenant au moins le nombre de fibres optiques utilisées, leur position par rapport à une grille de brachythérapie, et la longueur d'insertion de chacune des fibres dans la zone à traiter,
- et d'autre part, les volumes d'action théoriques calculés à partir du même ensemble de paramètres et du volume élémentaire d'action théorique d'une fibre.

Les volumes d'action théoriques étant fonction du volume élémentaire d'action théorique d'une fibre, qui lui est même est fonction notamment du rayon d'action, il est donc possible de déterminer très simplement ce rayon d'action.

En référence à la figure 2, le procédé 100 de modélisation comporte ainsi une étape préalable 110 de construction d'une base de données à partir des résultats d'essais cliniques effectués antérieurement sur une pluralité de patients en utilisant la même substance photosensible selon le même dosage, par exemple 4 mg/Kg de WST11, associée à au moins une fibre optique que l'on cherche à modéliser. Cette construction consiste à mémoriser dans la base de données un enregistrement par patient comportant au minimum les éléments suivants :

- un premier fichier numérique correspondant à une série d'images à résonance magnétique de la zone à traiter avant l'essai clinique, de préférence une série d'images transversales;
- un deuxième fichier numérique correspondant à une série d'images à résonance magnétique de la zone après l'essai clinique, de préférence quelques jours après le traitement de la zone par PDT ;
- l'ensemble des paramètres correspondant aux conditions réelles de réalisation de l'essai clinique, à savoir au moins le nombre de fibres optiques qui ont été utilisées, leur position par rapport à la grille de brachythérapie, et la longueur d'introduction de chacune des fibres dans la zone à traiter.

A titre d'exemple, la figure 4 représente schématiquement une coupe transversale d'une prostate de contour 3 avant traitement, les fibres optiques 4 qui ont été effectivement utilisées pour le traitement PDT de l'essai clinique considéré, à savoir douze fibres optiques identiques, ainsi que leur positionnement. Le point central 5 représente schématiquement la position de l'urètre. A côté de chaque point représentatif d'une fibre optique, un nombre indique la longueur d'insertion de chaque fibre dans la prostate. La figure 5 représente quant à elle la même coupe transversale de la prostate obtenue par une image à résonance magnétique prise sept jours après le traitement PDT.

Une fois la base de données créée, une étape 120 du procédé est mise en oeuvre de façon à déterminer par mesure, pour chaque patient de la base de données, à savoir pour chaque enregistrement de la base de données, le volume de la zone effectivement nécrosée lors de l'essai clinique, à partir desdits premier et deuxième fichiers numériques.

Selon une réalisation préférée de l'invention, cette étape 120 comprend avantageusement une première étape 121 lors de laquelle la série d'images du deuxième fichier informatique est chargée puis affichée, image par image, sur une interface graphique utilisateur (non représentée) visualisée sur un écran de l'ordinateur. L'utilisateur, en l'occurrence le radiologue ou le chirurgien, peut alors procéder, dans une étape 122, au contourage de la zone qui a effectivement été nécrosée par saisie directe sur chaque image de la série affichée sur l'écran d'ordinateur, de préférence par l'intermédiaire de la souris reliée à l'ordinateur. Le contour saisi par le praticien s'affiche directement en surimpression sur chacune des images, lui permettant d'apporter toutes les modifications de contour qui s'avéreraient nécessaire avant de passer à l'étape suivante. Le calcul du volume de la zone effectivement nécrosée peut alors intervenir dans une étape 123, par reconstruction volumique à partir d'un traitement numérique classique des contours saisis.

Les étapes 121 à 123 sont réitérées pour chaque enregistrement de la base de données. A l'issue de l'étape 120, on dispose ainsi de tous les volumes de zones effectivement nécrosées pour tous les essais cliniques mémorisés dans la base de données.

L'étape suivante 130 du procédé de l'invention, qui peut d'ailleurs être menée indifféremment avant, après ou même simultanément à l'étape 120, consiste alors au calcul, pour chaque patient de la base de données, du volume total théorique d'action en fonction du nombre total de fibres, de la position de chaque fibre, de la longueur d'insertion de chaque fibre et du volume élémentaire d'action théorique d'une fibre optique donné par la relation suivante :

$$V = \pi R^2 L$$

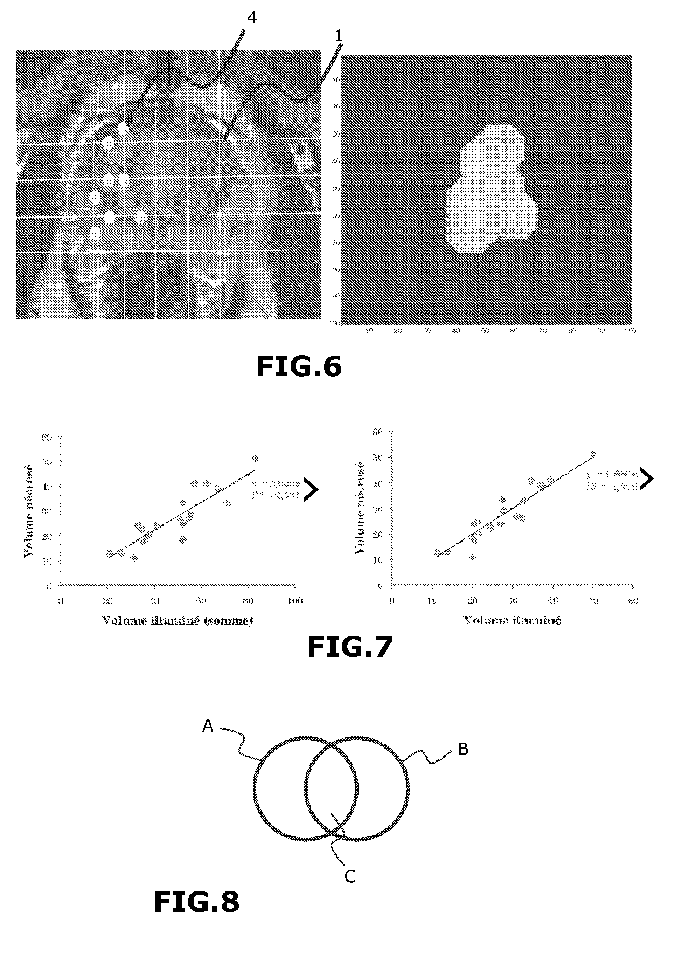

Ici encore, ce volume est calculé par des techniques classiques de reconstruction volumique, mais en prenant compte ici, dans chaque plan transversal, le contour global de l'ensemble des fibres optiques, chaque fibre optique étant assimilée à un cylindre. La figure 6 donne à titre d'exemple une comparaison entre une image à résonance magnétique transversale d'une prostate issue du deuxième fichier numérique sur laquelle apparait en surimpression la position des fibres optiques 4 effectivement utilisées lors de l'essai clinique correspondant, par rapport à la grille 1 de brachythérapie (partie gauche de la figure 6) et le contour global, dans ce même plan, lorsque le volume d'action de chaque fibre, représentée par un rectangle blanc, est modélisé par un cylindre (partie droite de la figure 6).

Une fois que les étapes 120 et 130 ont été réalisées, il ne reste plus qu'à procéder, lors d'une étape référencée 140 sur la figure 2, à la détermination du rayon d'action R d'une fibre optique par corrélation, pour chaque patient dans la base de données, du volume total théorique d'action calculé avec le volume mesuré de la zone effectivement nécrosée.

Le procédé de modélisation 100 décrit ci-dessus a été validé sur une base de données constituée à partir des résultats issus de 28 essais cliniques réalisés avec un dosage de 4 mg par kilo des patients concernés, en utilisant à chaque fois un nombre de fibres sur des longueurs d'insertion variant de 15 à 40 mm par pas de 5 mm.

La courbe représentée sur la partie droite de la figure 7 illustre la très bonne corrélation (indice de corrélation égal à 0,87) obtenue entre les volumes théoriques et les volumes des zones effectivement nécrosées suite aux essais cliniques. En recherchant la valeur de R pour laquelle le volume théorique est le plus proche du volume de la zone effectivement nécrosée pour les 28 essais cliniques, un rayon moyen de 7,49 mm, avec une précision de 1,08 mm, a été trouvé.

Le graphique sur la partie gauche de la figure 7 représente quant à lui les résultats d'une corrélation du volume effectivement nécrosé avec la somme des volumes élémentaires des fibres. On note donc qu'on obtient une bien meilleure corrélation en considérant le volume réel formé par toutes les fibres en fonction de leur position plutôt que la somme des volumes élémentaires des fibres, car cela permet de prendre en compte le fait que, lorsque deux fibres A et B sont positionnées de sorte à avoir une zone de recouvrement C, comme illustré à titre d'exemple sur la figure 8, le volume réel ne correspond pas à la somme des volumes élémentaires de deux fibres disjointes, mais bien à la somme de ces volumes élémentaires moins le volume commun. Autrement dit, l'action d'une fibre ne doit pas être considérée dans la modélisation dans une partie de volume déjà recouvert par l'action d'une autre fibre, car les cellules présentes dans la zone de recouvrement ne peuvent être nécrosées qu'une seule fois.

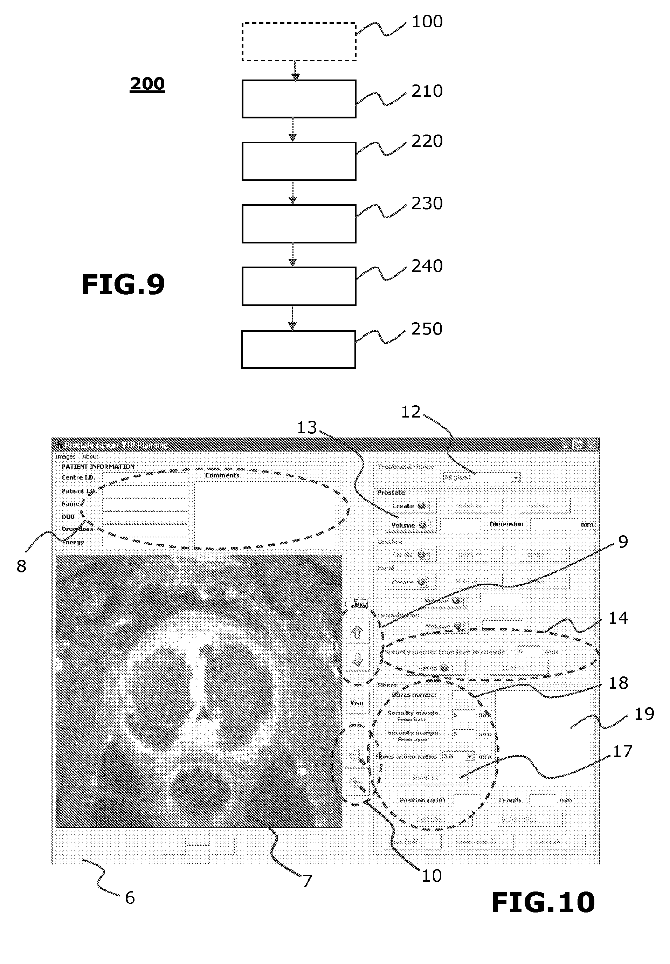

Les résultats de la modélisation décrite ci-dessus vont pouvoir à présent être utilisés pour la planification de tout futur traitement par PDT, en mettant en oeuvre le procédé 200 d'assistance à la planification selon l'invention qui va à présent être décrit, en référence notamment à la figure 9. Ici encore, le procédé d'assistance est destiné à être mis en oeuvre par un produit logiciel apte à être installé sur un ordinateur, et certaines étapes du procédé nécessitent l'intervention d'un utilisateur, typiquement le praticien, par l'intermédiaire d'une interface graphique apte à être affichée sur l'écran de l'ordinateur, et pilotée par le produit logiciel. L'objectif de cette planification est de pouvoir délivrer rapidement au praticien un ensemble de paramètres optimisés en termes de nombres de fibres à utiliser, de leur positionnement par rapport à une grille de brachythérapie, et de longueur d'insertion dans la zone à traiter, par rapport à tout patient devant subir un traitement par thérapie photodynamique.

Pour pouvoir mettre en oeuvre les différentes étapes 210 à 240 qui vont à présent être détaillées, une calibration du produit logiciel doit avoir préalablement été effectuée de manière à fixer la valeur du rayon d'action R d'une fibre optique. Ceci peut être réalisé en mémorisant la valeur R déterminée par ailleurs, ou en donnant la possibilité à l'utilisateur de choisir une valeur de R parmi un ensemble de valeurs possibles, par exemple l'ensemble de valeurs {5,5 mm ; 5,6 mm ; 6,5 mm ; 7,5 mm ; 8,5 mm}. De préférence cependant, comme illustré sur la figure 9, cette valeur R est déterminée au sein du même produit logiciel, en mettant en oeuvre les étapes du procédé de modélisation 100 décrit précédemment. Ainsi, il est possible de recalibrer à tout moment la valeur du rayon d'action en prenant en compte tout nouveau essai clinique.

Une première étape 210 du procédé d'assistance consiste alors à permettre le chargement et l'affichage d'un fichier numérique correspondant à une série d'images numériques, soit à résonance magnétique, soit par ultrasons, de préférence transversales planes, de la zone à traiter sur une interface graphique utilisateur affichée sur un écran de l'ordinateur. Le fichier numérique est par exemple au format DICOM. La figure 10 illustre, à titre d'exemple une copie d'écran d'ordinateur représentant l'interface graphique utilisateur 6 comprenant une zone dans laquelle une image transversale 7 d'une prostate d'un patient a été chargée pour affichage. Comme on peut le voir sur cette copie d'écran, l'interface graphique 6 comporte également une zone 8 d'informations avec plusieurs champs que l'utilisateur peut renseigner par saisie directe au moyen d'un clavier relié à l'ordinateur, et différents boutons de commande permettant à l'utilisateur de déclencher plusieurs actions. Notamment, à l'issue de l'étape 210, l'utilisateur peut naviguer entre les différentes coupes du fichier numérique d'images grâce aux boutons de navigation d'une zone 9 de l'interface, zoomer, respectivement dé-zoomer sur chaque image 7 affichée par l'intermédiaire de deux boutons d'une zone 10 de l'interface. D'autres boutons permettant le déplacement de l'image affichée, ou la modification du contraste. Diverses informations sur le patient peuvent être saisies directement dans la zone 8 de l'interface, par exemple le centre d'examen, l'identifiant et le nom du patient, sa date de naissance, la dose et l'énergie délivrées, et tout commentaire approprié. Toutes ces informations sont sauvegardées au terme de la planification, par exemple dans un fichier au format PDF.

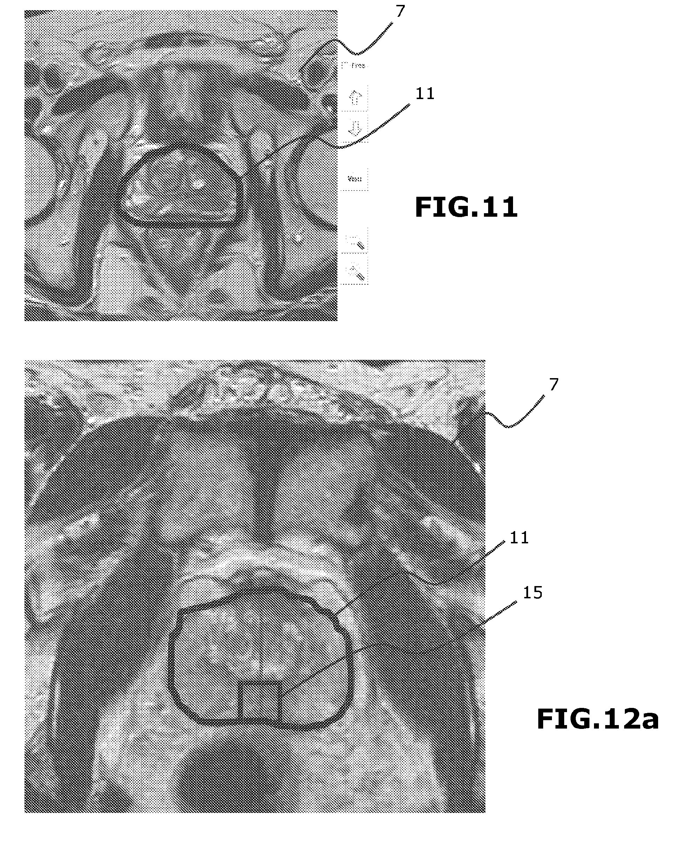

A l'issue de l'étape 210, le praticien peut procéder, directement sur l'interface graphique 6, à une étape 220 de contourage de la zone à traiter. Ce contourage s'effectue par saisie directe sur chaque image 7 de la série affichée sur l'interface graphique 6, de préférence par l'intermédiaire de la souris reliée à l'ordinateur. Le contour saisi par le praticien s'affiche directement en surimpression sur chacune des images, lui permettant d'apporter toutes les modifications de contour qui s'avéreraient nécessaire avant de passer à l'étape suivante. La figure 11 représente, à titre d'exemple, une copie d'écran d'ordinateur représentant la zone de l'interface graphique utilisateur dans laquelle on peut visualiser un contour 11 dessiné par l'utilisateur apparaît en surimpression de l'image transversale plane 7.

Il convient de noter à ce stade que, lors d'un traitement par PDT du cancer de la prostate, différentes zones de la glande peuvent être traitées. En conformité avec ces différentes procédures réalisées en clinique, le procédé d'assistance mis en oeuvre par ordinateur permet avantageusement à l'utilisateur de définir celle appropriée au patient. Il offre ainsi quatre possibilités :

- Le traitement de la glande entière ;
- Le traitement du lobe droit (hémiablation droite) ;
- Le traitement du lobe gauche (hémiablation gauche) ;
- Le traitement focal.

Préalablement à l'étape de contourage 220, l'utilisateur peut choisir avantageusement le type de traitement qu'il envisage, par exemple par l'intermédiaire d'une zone de sélection 12 par menu déroulant de l'interface graphique utilisateur (voir figure 10).

En fonction du type de traitement sélectionné, l'utilisateur pourra avoir à saisir, outre le contour de la prostate, d'autres contours définissant plus précisément la zone à traiter.

A l'issue de l'étape de contourage 220, on dispose ainsi d'une série d'IRM transversales et de contours de la zone à traiter apparaissant en surimpression sur la zone d'affichage de l'interface graphique utilisateur. Les mêmes boutons de navigation 9, de grossissement 10, de modification de contraste ou de déplacement que ceux décrits précédemment peuvent être utilisés à ce stade. Les contours peuvent avantageusement être sauvegardés pour être rechargés et affichés ultérieurement à tout moment.

L'étape suivante 230 du procédé d'assistance à la planification selon l'invention consiste alors en la mesure du volume de la zone à traiter par reconstruction volumique à partir d'un traitement numérique classique des contours saisis à l'étape 220. Cette étape est de préférence déclenchée suite à l'action de l'utilisateur sur un bouton de commande spécifique, tel que le bouton de commande 13 représenté sur la figure 10, dans le cas où la zone à traiter correspond à l'ensemble de la prostate. Le volume ainsi mesuré est avantageusement affiché en cm$^3$ sur l'interface graphique. Lorsque le volume mesuré est celui de la prostate, on peut prévoir également de calculer et d'afficher les dimensions maximales en millimètres de la prostate dans les trois plans de l'espace (transversal, sagittal et coronal).

L'étape 240 suivante du procédé d'assistance à la planification selon l'invention consiste à permettre l'affichage et le positionnement d'une représentation plane de la grille de brachythérapie en surimpression sur chaque image de la série et contours saisis correspondants. Pour ce faire, l'utilisateur doit sélectionner une image, parmi la série d'images transversale préalablement chargée, de préférence celle correspondant à la coupe centrale de la série. Cette image sélectionnée est affichée dans la zone d'affichage correspondante de l'interface graphique, avec, en surimpression, le ou les contours préalablement saisis à l'étape 220. A ce stade, l'utilisateur peut avantageusement définir une première marge de sécurité correspondant à la distance minimale nécessaire entre les positions des fibres éligibles et la capsule de la prostate. Cette distance est par défaut initialisée à une valeur fixe prédéterminée, par exemple égale à 6 mm, mais elle peut être modifiée par l'intermédiaire de l'interface graphique, au niveau d'une zone de saisie 14 (voir figure 10). L'étape 240 est de préférence déclenchée par l'utilisateur, par exemple par actionnement d'un bouton d'action spécifique de l'interface graphique utilisateur situé dans la zone de saisie 14.

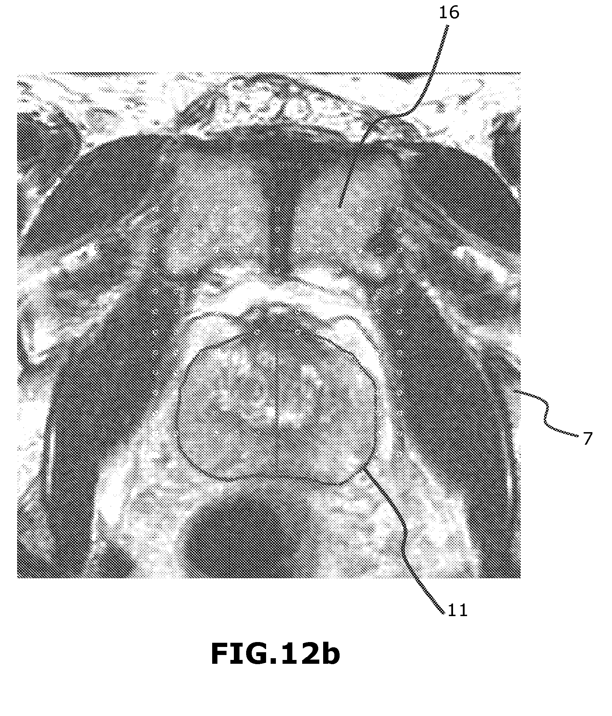

Les figures 12a et 12b représentent, à titre d'exemple, deux copies d'écran obtenues lors de la mise en oeuvre de l'étape 240. Plus précisément, la figure 12a représente la zone de l'interface graphique utilisateur sur laquelle apparaissent simultanément l'image transversale plane 7 sélectionnée, le contour 11 associé préalablement saisi par l'utilisateur (étape 220), et un carré 15 dont le côté correspond à la première marge de sécurité, et dont le centre correspond à une position spécifique sur la grille de brachythérapie, typiquement la position D1 correspondant à l'intersection entre la colonne centrale D de la grille illustrée sur la figure 1, et la ligne inférieure 1 de cette grille. Ce carré peut être avantageusement déplacé par l'utilisateur par l'intermédiaire de l'interface graphique en cliquant sur son point central.

La figure 12b représente quant à elle la zone de l'interface graphique 6 sur laquelle apparaît une représentation plane graphique 16 de la grille de brachythérapie, en surimpression par rapport à une image transversale plane 7 sélectionnée et à un contour 11 correspondant préalablement saisi (étape 220). La représentation plane 16 apparaît comme une pluralité de signes, ici des cercles, disposés en matrice selon la grille de brachythérapie de la figure 1. Des couleurs peuvent avantageusement être utilisées à ce stade pour donner une indication visuelle à l'utilisateur des positions de fibres éventuellement éligibles pour le traitement. Par exemple, un cercle de couleur blanche signifiera que la position n'est pas éligible car située en dehors du contour de la prostate, et un cercle ce couleur rouge correspondra à une position à l'intérieur du contour de la prostate, mais non éligible soit, parce qu'elle n'est pas dans la zone à traiter, soit parce qu'elle est trop proche d'une zone à risque, par exemple de la capsule ou de l'urètre, en fonction de la marge de sécurité préalablement définie. Un cercle de couleur verte pourra alors signifier qu'une position particulière est éligible, ce qui donne ainsi une indication sur le nombre maximum de fibres optiques qui pourra être choisi.

L'étape suivante 250 (figure 9) constitue le coeur du procédé d'assistance à la planification selon l'invention. Elle concerne la détermination automatique par le calcul des paramètres optimaux en terme de nombre de fibres optiques à utiliser, de leur position par rapport à la grille de brachythérapie et de leur longueur d'insertion qui vont permettre d'obtenir la meilleure correspondance entre un volume total théorique d'action calculé avec le volume mesuré à l'étape 230 de la zone à traiter.

Le calcul du volume théorique d'action repose sur les mêmes principes de modélisation que ceux explicités en référence au procédé de modélisation 100 de la figure 2. Ainsi, ce volume total théorique d'action est calculé en fonction de la position de chaque fibre, et du volume élémentaire d'action théorique d'une fibre, ce dernier volume élémentaire correspondant au volume d'un cylindre de rayon d'action R prédéterminé, et de hauteur correspondant à la longueur d'insertion de la fibre.

Pour permettre l'optimisation, l'étape 250 utilise de préférence un algorithme d'optimisation de type descente de gradient, comme l'algorithme de Powell. Cet algorithme réalise des minimisations unidimensionnelles suivant des directions conjuguées. Deux vecteurs (ou directions) $s_1$ et $s_2$ de $\mathbb{R}^n$ sont conjugués vis-à-vis d'une matrice définie positive et symétrique A si $s_1^T A s_2 = 0$. Cet algorithme nécessite ainsi la définition d'une fonction objective f à minimiser. Ici, la fonction f a été définie de façon que l'algorithme positionne les fibres au mieux, c'est-à-dire avec un recouvrement optimal de la zone cible, mais sous la contrainte qu'il ne faut pas de région nécrosée extra-prostatique (en dehors de la cible). La fonction utilisée peut être définie mathématiquement par la relation suivante :

$$f = \sum_{i=1}^{N} w_1 v_i \cap T - w_2 v_i \cap H$$

Dans laquelle N est un nombre total de fibres, i est un indice représentatif d'une fibre spécifique, v est un voxel, T la région cible et H celle restante en dehors de la cible, et $w_1$ et $w_2$ sont des poids positifs fixés.

Autrement dit, l'algorithme de Powell est ici utilisé pour trouver les meilleures valeurs de paramètres (nombre N, position de chaque fibre i, longueur d'insertion) qui vont minimiser la différence entre le volume théorique calculé pour différentes valeurs de paramètres et le volume mesuré de la zone à traiter. En pratique, l'algorithme va débuter en imposant une première valeur de N possible, estimée en divisant le volume cible mesuré à l'étape 230 par le volume élémentaire d'action théorique d'une fibre, ce dernier étant égal à $\pi R^2 L$, où R est le rayon d'action prédéfini de la fibre.

L'algorithme se poursuit alors en recherchant de manière itérative, pour toutes les positions et longueur d'insertion possibles de fibres, celles qui permettront à l'algorithme de converger vers la valeur minimale de la fonction f. Compte tenu de la modélisation choisie et de la simplicité des calculs mis en oeuvre par l'algortithme, un résultat peut être obtenu très rapidement, au bout d'une durée de quelques minutes seulement. Les calculs sont d'autant plus rapides que l'algorithme ne considèrera que les positions effectivement éligibles (cercles de couleur verte sur la représentation plane de la grille affichée à l'étape 240).

L'étape d'optimisation 250 est de préférence déclenchée par l'utilisateur, par exemple par actionnement d'un bouton d'action spécifique 17 de l'interface graphique utilisateur 6 situé dans une zone de saisie 18 (voir figure 10).

Il convient de noter qu'une plus grande souplesse d'utilisation peut être également offerte en proposant à l'utilisateur, avant le lancement de l'optimisation 250, de choisir certains des paramètres, comme par exemple le nombre de fibres à utiliser, pour autant que ce nombre ne soit pas incompatible avec le nombre maximum de fibres qui sont éligibles dans la zone à traiter. Dans ce cas, le nombre de fibres est saisi directement par l'utilisateur, de préférence au niveau de la zone de saisie 18 de l'interface graphique. Par ailleurs, l'utilisateur peut définir lui-même, en plus de la première marge de sécurité précitée, deux marges de sécurité supplémentaires, une première marge qui correspond à la distance minimale entre l'extrémité des fibres et la capsule de la prostate au niveau de la base (fixée par défaut à 5 mm), et une deuxième marge de sécurité qui correspond à la distance minimale entre l'extrémité des fibres et la capsule de la prostate au niveau de l'apex (fixée par défaut à 3 mm). Ces différentes marges de sécurité peuvent avantageusement être modifiées par l'utilisateur, par saisie directe, au niveau de champs spécifiquement dédiés dans la zone de saisie 18 de l'interface graphique. Enfin, comme cela a déjà été mentionné précédemment, l'utilisateur peut sélectionner la valeur R du rayon d'action dans l'ensemble de valeurs {5,5 mm ; 5,6 mm ; 6,5 mm ; 7,5 mm ; 8,5 mm}.

Dans tous les cas, à la fin de l'étape 250 d'optimisation, on dispose d'un résultat donnant au minimum le nombre (choisi par l'utilisateur ou optimisé automatiquement), la position et la longueur de chaque fibre, la valeur de tous ces paramètres ayant été optimisée au cas particulier du patient. Ce résultat est avantageusement affiché sur l'interface graphique de l'utilisateur, par exemple dans une zone d'affichage spécifique 19 (figure 10) proche de la zone de saisie 18.

En plus de ces données, on peut prévoir également d'afficher les données additionnelles suivantes :

- le taux de recouvrement de la nécrose simulé par rapport au volume cible.
- un indice correspondant à la somme des longueurs des fibres sur le volume cible.
- la somme des longueurs des fibres.
- Une représentation graphique du volume d'action simulé sur les images IRM dans les trois incidences (axial - coronal et sagittal).

Bien entendu, le résultat est également mémorisé, par exemple sous la forme d'un fichier au format PDF, de manière à ce que l'utilisateur puisse consulter à tout moment, par affichage sur ordinateur ou impression sur papier, d'un rapport de planification donnant un récapitulatif notamment :

- de toute information du patient préalablement entrée sur l'interface graphique ;

- des différents volumes mesurés ;

- du type de traitement choisi ;

- des marges de sécurité considérées dans l'optimisation ;

- du rayon d'action des fibres utilisé ;

- du nombre de fibres choisi ou optimisé ;

- de la position et la longueur des fibres ;

- des distances entre chaque fibre

- de l'indice, de la somme des longueurs des fibres et du taux de recouvrement obtenus.

- des différentes coupes IRM utilisées pour la planification.

La planification obtenue peut être avantageusement affinée, par exemple en venant modifier manuellement le nombre de fibres. A chaque ajout ou suppression de fibre, le taux de recouvrement, l'indice et la somme des longueurs des fibres sont recalculés et affichés sur l'interface. Une nouvelle planification pour un même patient peut en outre être facilement lancée en modifiant un ou plusieurs paramètres choisis parmi :

- le positionnement de la grille de brachythérapie

- le nombre de fibres

- les marges de sécurité

- le rayon d'action des fibres

**Revendications**

1. Procédé (100) de modélisation, mis en oeuvre par ordinateur, de l'action d'une fibre optique (4) destinée à être utilisée dans un traitement d'un patient par thérapie photodynamique pour illuminer, à une longueur d'onde prédéfinie, une zone à traiter sur une longueur d'insertion de la fibre optique à l'intérieur de ladite zone à traiter, afin d'activer une substance photosensible prédéfinie préalablement administrée audit patient et présente dans ladite zone à traiter, **caractérisé en ce qu'**il consiste à modéliser le volume élémentaire d'action théorique de la fibre optique par celui d'un cylindre de rayon d'action R et de longueur L correspondant à ladite longueur d'insertion, et à déterminer (140) ledit rayon d'action R par corrélation de volumes mesurés de zones effectivement nécrosées suite à une pluralité d'essais cliniques menés sur différents patients en utilisant ladite substance photosensible associée à au moins une fibre optique, chaque essai clinique étant associé à un ensemble de paramètres correspondant aux conditions réelles de l'essai clinique et comprenant au moins le nombre de fibres optiques utilisées, leur position par rapport à une grille de brachythérapie (1), et la longueur d'insertion de chacune des fibres dans la zone à traiter, avec des volumes d'action théoriques calculés à partir du même ensemble de paramètres et du volume élémentaire d'action théorique.

**2.** Procédé (100) de modélisation selon la revendication 1, **caractérisé en ce qu'**il comporte les étapes suivantes :

- Construction (110) d'une base de données informatique à partir desdits essais cliniques, par mémorisation pour chaque patient, dans ladite base de données, d'un premier fichier numérique correspondant à une série d'images numériques de la zone à traiter avant l'essai clinique, d'un deuxième fichier numérique correspondant à une série d'images numériques de la zone après l'essai clinique, et dudit ensemble de paramètres correspondant aux conditions réelles de l'essai clinique;
- Mesure (120), pour chaque patient de la base de données, du volume de la zone effectivement nécrosée lors de l'essai clinique, à partir desdits premier et deuxième fichiers numériques ;
- Calcul (130), pour chaque patient de la base de données, du volume total théorique d'action en fonction des paramètres dudit ensemble et du volume élémentaire d'action théorique d'une fibre;
- Détermination (140) dudit rayon d'action R d'une fibre optique par corrélation du volume total théorique d'action calculé, pour chaque patient dans la base de données, avec le volume mesuré de la zone effectivement nécrosée.

**3.** Procédé (100) de modélisation selon la revendication 1, **caractérisé en ce que** les séries d'images numériques desdits premier et deuxième fichiers numériques correspondent à des images transversales de la zone respectivement avant et après traitement.

**4.** Procédé (100) de modélisation selon l'une quelconque des revendications précédentes, **caractérisé en ce que** lesdites images numériques sont des images à résonance magnétique ou des images par ultrasons.

**5.** Procédé (100) de modélisation selon l'une quelconque des revendications précédentes, **caractérisé en ce que** l'étape (120) de mesure du volume de la zone effectivement nécrosée comporte les étapes suivantes :

- Chargement et affichage (121) de la série d'images du deuxième fichier informatique sur une interface graphique utilisateur affichée sur un écran de l'ordinateur;
- Contourage (122) de la zone effectivement nécrosée par saisie directe sur chaque image de la série affichée sur l'écran d'ordinateur;
- Mesure (123) du volume de la zone effectivement nécrosée par reconstruction volumique à partir d'un traitement numérique des contours saisis.

**6.** Procédé (200) d'assistance, mis en oeuvre par ordinateur, à la planification d'un traitement d'un patient par thérapie photodynamique, lors duquel une substance photosensible prédéfinie doit être administrée au patient, puis soumise à une illumination à une longueur d'onde prédéterminée par l'intermédiaire d'un nombre de fibres optiques aptes à être introduites sur une longueur d'insertion dans la zone à traiter selon une position par rapport à une grille de brachythérapie (1), **caractérisé en ce qu'**il comporte les étapes suivantes:

- Chargement et affichage (210) d'un fichier numérique correspondant à une série d'images (7) numériques de la zone à traiter sur une interface graphique utilisateur (6) affichée sur un écran de l'ordinateur;
- Contourage (220) de la zone à traiter par saisie directe sur chaque image de la série affichée sur l'écran d'ordinateur;
- Mesure (230) du volume de la zone à traiter par reconstruction volumique à partir d'un traitement numérique des contours (11) saisis ;
- Affichage et positionnement (240) d'une représentation plane (16) de la grille de brachythérapie (1) en surimpression sur chaque image (7) de la série et contours (11) saisis correspondants ;
- Détermination (250) par le calcul du nombre de fibres optiques à utiliser, de leur position par rapport à la grille de brachythérapie et de leur longueur d'insertion qui optimisent la correspondance d'un volume total théorique d'action calculé avec le volume mesuré de la zone à traiter, ledit volume total théorique d'action étant calculé en fonction de la position de chaque fibre, et d'un volume élémentaire d'action théorique d'une fibre correspondant au volume d'un cylindre de rayon d'action R prédéterminé, et de hauteur correspondant à la longueur d'insertion de la fibre.

**7.** Procédé (200) d'assistance selon la revendication 6, **caractérisé en ce que** ledit rayon d'action R est prédéterminé conformément au procédé de modélisation selon l'une quelconque des revendications 1 à 5.

**8.** Procédé (200) d'assistance selon la revendication 6, **caractérisé en ce que** ledit rayon d'action R est sélectionné parmi un ensemble de valeurs possibles.

**9.** Procédé (200) d'assistance selon l'une quelconque des revendications 6 à 8, **caractérisé en ce que** ladite série d'images (7) numériques de la zone à traiter correspond à des images transversales de la zone à traiter.

**10.** Procédé (200) d'assistance selon l'une quelconque des revendications précédentes, **caractérisé en ce que** lesdites images numériques sont des images à résonance magnétique ou des images par ultrasons.

**11.** Procédé (200) d'assistance selon l'une quelconque des revendications 6 à 10, **caractérisé en ce que** l'étape (250) de détermination par le calcul utilise un algorithme d'optimisation de type descente de gradient.

**12.** Procédé (200) d'assistance selon la revendication 11, **caractérisé en ce que** l'étape (250) de détermination par le calcul utilise l'algorithme de Powell.

**13.** Produit programme d'ordinateur qui, lorsqu'il est mis en oeuvre sur un ordinateur informatique, réalise le procédé de modélisation selon l'une quelconque des revendications 1 à 5.

**14.** Produit programme d'ordinateur qui, lorsqu'il est mis en oeuvre sur un ordinateur informatique, réalise le procédé d'assistance selon l'une quelconque des revendications 6 à 12.

**FIG.1**

**FIG.2**

## Profil d'émission de la fibre M30 sous différents angles d'observation - 763nm

**FIG.3**

**FIG.4**

**FIG.5**

**FIG.6**

**FIG.7**

**FIG.8**

**200**

**FIG.9**

**FIG.10**

**FIG.11**

**FIG.12a**

**FIG.12b**

Europäisches
Patentamt

European
Patent Office

Office européen
des brevets

# RAPPORT DE RECHERCHE EUROPEENNE

Numéro de la demande

EP 10 30 5960

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | JERZY JANKUN, RICK W. KECK, EWA SKRZYPCZAK-JANKUN, LOTHAR LILGE, STEVEN H. SELMAN: "Diverse optical characteristic of the prostate and light delivery system: implications for computer modelling of prostatic photodynamic therapy", BJU INTERNATIONAL, vol. 95, 12 mai 2005 (2005-05-12), pages 1237-1244, XP002612679, DOI: 10.1111/j.1464-410X.2005.05512.x * le document en entier * | 13 | INV. A61N5/06 |
| A | WO 2010/089416 A1 (SPECTRACURE AB [SE]; SWARTLING JOHANNES [SE]) 12 août 2010 (2010-08-12) * abrégé * * page 19, ligne 3-37 * | 13 | |
| A,D | SEAN R H DAVIDSON ET AL: "Treatment planning and dose analysis for interstitial photodynamic therapy of prostate cancer; Treatment planning and analysis for prostate PDT", PHYSICS IN MEDICINE AND BIOLOGY, TAYLOR AND FRANCIS LTD. LONDON, GB, vol. 54, no. 8, 21 avril 2009 (2009-04-21), pages 2293-2313, XP020149927, ISSN: 0031-9155, DOI: DOI:10.1088/0031-9155/54/8/003 * le document en entier * | 13 | DOMAINES TECHNIQUES RECHERCHES (IPC) A61N |

-/--

Le présent rapport a été établi pour toutes les revendications

| Lieu de la recherche | Date d'achèvement de la recherche | Examinateur |
|---|---|---|
| Munich | 9 mars 2011 | Grochol, Jana |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

| | Europäisches<br>Patentamt<br>European<br>Patent Office<br>Office européen<br>des brevets | **RAPPORT DE RECHERCHE EUROPEENNE** | Numéro de la demande<br>EP 10 30 5960 |
|---|---|---|---|

## DOCUMENTS CONSIDERES COMME PERTINENTS

| Catégorie | Citation du document avec indication, en cas de besoin,<br>des parties pertinentes | Revendication<br>concernée | CLASSEMENT DE LA<br>DEMANDE    (IPC) |
|---|---|---|---|
| X | AUGUSTO RENDON ET AL:  "Treatment planning using tailored and standard cylindrical light diffusers for photodynamic therapy of the prostate; Treatment planning with tailored and standard diffusers",<br>PHYSICS IN MEDICINE AND BIOLOGY, TAYLOR AND FRANCIS LTD. LONDON, GB,<br>vol. 53, no. 4,<br>21 février 2008 (2008-02-21), pages 1131-1149, XP020127382,<br>ISSN: 0031-9155<br>* le document en entier *<br>-----| 6,8-12, 14 | |
| | | | **DOMAINES TECHNIQUES RECHERCHES    (IPC)** |

Le présent rapport a été établi pour toutes les revendications

| Lieu de la recherche | Date d'achèvement de la recherche | Examinateur |
|---|---|---|
| Munich | 9 mars 2011 | Grochol, Jana |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons

......................................................................................
& : membre de la même famille, document correspondant

EPO FORM 1503 03.82 (P04C02)

Europäisches
Patentamt

European
Patent Office

Office européen
des brevets

## REVENDICATIONS DONNANT LIEU AU PAIEMENT DE TAXES

La présente demande de brevet européen comportait lors de son dépôt les revendications dont le paiement était dû.

☐ Une partie seulement des taxes de revendication ayant été acquittée dans les délais prescrits, le présent rapport de recherche européenne a été établi pour les revendications pour lesquelles aucun paiement n'était dû ainsi que pour celles dont les taxes de revendication ont été acquittées, à savoir les revendication(s):

☐ Aucune taxe de revendication n'ayant été acquittée dans les délais prescrits, le présent rapport de recherche européenne a été établi pour les revendications pour lesquelles aucun paiement n'était dû.

## ABSENCE D'UNITE D'INVENTION

La division de la recherche estime que la présente demande de brevet européen ne satisfait pas à l'exigence relative à l'unité d'invention et concerne plusieurs inventions ou pluralités d'inventions, à savoir:

voir feuille supplémentaire B

☒ Toutes les nouvelles taxes de recherche ayant été acquittées dans les délais impartis, le présent rapport de recherche européenne a été établi pour toutes les revendications.

☐ Comme toutes les recherches portant sur les revendications qui s'y prêtaient ont pu être effectuées sans effort particulier justifiant une taxe additionnelle, la division de la recherche n'a sollicité le paiement d'aucune taxe de cette nature.

☐ Une partie seulement des nouvelles taxes de recherche ayant été acquittée dans les délais impartis, le présent rapport de recherche européenne a été établi pour les parties qui se rapportent aux inventions pour lesquelles les taxes de recherche ont été acquittées, à savoir les revendications:

☐ Aucune nouvelle taxe de recherche n'ayant été acquittée dans les délais impartis, le présent rapport de recherche européenne a été établi pour les parties de la demande de brevet européen qui se rapportent à l'invention mentionnée en premier lieu dans les revendications, à savoir les revendications:

☐ Le present rapport supplémentaire de recherche européenne a été établi pour les parties de la demande de brevet européen qui se rapportent a l'invention mentionée en premier lieu dans le revendications (Règle 164 (1) CBE)

Europäisches
Patentamt

European
Patent Office

Office européen
des brevets

**ABSENCE D'UNITÉ D'INVENTION
FEUILLE SUPPLÉMENTAIRE B**

Numéro de la demande

EP 10 30 5960

La division de la recherche estime que la présente demande de brevet européen ne satisfait pas à l'exigence relative à l'unité d'invention et concerne plusieurs inventions ou pluralités d'inventions, à savoir :

```
1. revendications: 1-5, 13

        procédé de modélisation de l'action d'une fibre optique
        destinée à être utilisée dans un traitement d'un patient par
        thérapie photodynamique
                            ---

2. revendications: 6-12, 14

        procédé d'assistance à la planification d'un traitement d'un
        patient par thérapie photodynamique
                            ---
```

EPO FORM P0402

**EP 2 425 873 A1**

**ANNEXE AU RAPPORT DE RECHERCHE EUROPEENNE
RELATIF A LA DEMANDE DE BREVET EUROPEEN NO.**

EP 10 30 5960

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

09-03-2011

| Document brevet cité au rapport de recherche | Date de publication | Membre(s) de la famille de brevet(s) | Date de publication |
|---|---|---|---|
| WO 2010089416 A1 | 12-08-2010 | AUCUN | |

Pour tout renseignement concernant cette annexe : voir Journal Officiel de l'Office européen des brevets, No.12/82

# EP 2 425 873 A1

## RÉFÉRENCES CITÉES DANS LA DESCRIPTION

**Documents brevets cités dans la description**

- WO 2004045492 A

- EP 1137411 A

**Littérature non-brevet citée dans la description**

- **SEAN R H DAVIDSON et al.** Treatment and planning and dose analysis for interstitial photodynamic therapy of prostate cancer. *Phys. Med. Biol.,* 2009, vol. 54, 2293-2313